# EUROPÄISCHE PATENTSCHRIFT

(11) **EP 1 174 517 B1**
(45) Veröffentlichungstag und Bekanntmachung des Hinweises auf die Patenterteilung: **06.04.2005**
(21) Anmeldenummer: 01115693.2
(22) Anmeldetag: 05.07.2001
(51) Int. Cl.: A01H 3/04, C12N 9/10, C12N 15/82, C12Q 1/48, G01N 33/68

(54) **Verwendung von Beta-Ketoacyl-CoA-Synthase zum Auffinden von Herbiziden**
Use of Beta-Ketoacyl-CoA-Synthase for the screening of herbicides
Utilisation de la Beta-Ketoacyl-CoA-Synthase pour l'identification des herbicides

(30) Priorität: 18.07.2000 DE 10034804
(43) Veröffentlichungstag der Anmeldung: 23.01.2002
(73) Patentinhaber: Bayer CropScience AG, 40789 Monheim (DE)
(72) Erfinder: Lechelt-Kunze, Christa, Dr., 50935 Köln (DE); Meissner, Ruth, Dr., 51381 Leverkusen (DE); Tietjen, Klaus, Dr., 40764 Langenfeld (DE)

(56) Entgegenhaltungen:
- WO-A-98/54954
- BÖGER PETER ET AL: "Towards the primary target of chloroacetamides: New findings pave the way." PEST MANAGEMENT SCIENCE, Bd. 56, Nr. 6, Juni 2000 (2000-06), Seiten 497-508, XP001074233 ISSN: 1526-498X
- SCHMALFUSS JOCHEN ET AL: "Inhibition of acyl-CoA elongation by chloroacetamide herbicides in microsomes from leek seedlings." PESTICIDE BIOCHEMISTRY AND PHYSIOLOGY, Bd. 67, Nr. 1, Mai 2000 (2000-05), Seiten 25-35, XP001097160 ISSN: 0048-3575
- ABULNAJA K O ET AL: "INHIBITION OF FATTY ACID ELONGATION PROVIDES A BASIS FOR THE ACTION OF THE HERBICIDE ETHOFUMESATE ON SURFACE WAX FORMATION" PHYTOCHEMISTRY (OXFORD), Bd. 31, Nr. 4, 1992, Seiten 1155-1159, XP008006655 ISSN: 0031-9422
- MÖLLERS C ET AL: "Screening herbicide effects on lipid metabolism of storage lipids by in vitro culture of microspore-derived embryoids of Brassica napus." JOURNAL OF PLANT PHYSIOLOGY, Bd. 144, Nr. 3, 1994, Seiten 376-384, XP008006600 ISSN: 0176-1617
- BAZOOBANDI M ET AL: "Analysis of flufenacet in soil, wheat grain and straw by gas chromatography" JOURNAL OF CHROMATOGRAPHY, ELSEVIER SCIENCE PUBLISHERS B.V. AMSTERDAM, NL, Bd. 886, Nr. 1-2, Juli 2000 (2000-07), Seiten 319-322, XP004205994 ISSN: 0021-9673

## Beschreibung

Die Erfindung betrifft die Verwendung von Nukleinsäuren, die für pflanzliche Polypeptide mit der biologischen Aktivität einer Beta-Ketoacyl-CoA-Synthase kodieren und die Verwendung der davon kodierten Polypeptide zum Identifizieren von neuen, herbizid wirksamen Verbindungen.

Fettsäure-Elongasen werden auch als 3-Ketoacyl-CoA-Synthasen oder als "condensing enzymes" bezeichnet. Very Long Chain Fatty Acid Elongasen, im weiteren als VLCFAE abgekürzt, sind verantwortlich für die Biosynthese von Fettsäuren mit einer Kettenlänge von mehr als 18 C-Atomen (Millar, A.A. und Kunst, L. 1997, The Plant Journal 12(1), 121-131). VLCFAE erkennen als Substrate neben Malonyl-CoA-Ester nur Fettsäure-CoA-Ester mit Kettenlängen größer/gleich C₁₈ (C18+) und sind im Cytoplasma wahrscheinlich mikrosomal assoziierte Proteine. Die pflanzliche *de novo* Fettsäuresynthese bis 18C-Atomen verläuft im Chloroplasten und die Fettsäuren sind dabei mit einem Acyl Carrier Protein (ACP) verestert (WO 98/54954). Langkettige Fettsäuren, im weiteren Verlauf als VLCFA bezeichnet, sind Komponenten und Vorstufen der Wachsschicht in der Kutikula von Pflanzen. Sie können Komponenten und Vorstufen der Zellmembranen sein oder Bestandteile spezieller Speicher-Triacylglyceride. Sie können aber auch Bestandteile oder Vorstufen von Lipiden mit Signalfunktion oder mit "second messenger"-Funktion in der Pflanze darstellen, wie z.B. die Sphingolipide. (Millar, A.A. und Kunst, L. 1997, The Plant Journal 12(1), 121-131, Millar et al. 2000, Trends in Plant Science 5, 95-101).

Aus biochemischen Arbeiten (Matthes et al., Z. Naturforsch. 1998, 53c, 1004-1011, Abulnaja und Harwood, Phytochemistry 1991, 30(5), 1445-1447) ist bekannt, dass herbizide Substanzklassen wie z.B. Chloroacetamide, Oxyacetamide und Thiocarbamate die Synthese langkettiger Fettsäuren hemmen. In diesen Arbeiten ist allerdings nicht gezeigt, ob dies durch direkte Hemmung von bzw. Interaktion mit Fettsäure Elongase Enzymen geschieht. Ebenso ist unklar, welche der vielen in Pflanzen vorkommenden Elongasen gehemmt werden. Aus biochemischen und zellbiologischen Arbeiten ist ferner bekannt, dass Verbindungen wie BAS 13-338 die Fettsäure-Elongation von C18:1 hemmen (Möller und Albrecht 1994, J. Plant Physiol. 144, 376-384). Auch in dieser Arbeit sind die beteiligten Elongasen unbekannt.

VLCFAE sind pflanzenspezifische Gene, die zum Beispiel in Arabidopsis eine komplexe Genfamilie darstellen. Ein Gen dieser Familie, fiddlehead, wird spezifisch in der Epidermis, der äußersten Pflanzenzellschicht, exprimiert und unterdrückt die Dedifferenzierung dieser Zellen. Mutanten, in denen die Funktion des FIDDLEHEAD-Proteins gestört ist, zeigen Fusionen/Adhäsionen von Blättern und Blütenorganen. Die Fusionen/Adhäsionen der Blütenorgane einer Mutante *fiddlehead* ähneln der Schnecke einer Geige und gaben der Mutante diesen Namen. *Fiddlehead*-Pflanzen zeigen erhebliche morphologische Veränderungen und starke Entwicklungsstörungen. (Lolle et al. 1992, Developmental Biology 152, 383-392, Yephremov et al. 1999, The Plant Cell 11, 2187-2201, Pruitt et al. 2000, PNAS 97(3), 1311-1316). An der Fusion/Adhäsion sind im Wesentlichen Epidermiszellen beteiligt. Dabei fusionieren die Zellen nur über die extrazelluläre Schicht, das Cytoplasma verschmilzt dabei nicht. Da Epidermiszellen für die Bildung der extrazellulären Kutikula verantwortlich sind, ist anzunehmen, dass der Funktionsausfall von fiddlehead zu einer Veränderung der VLCFA Komponenten in der Kutikula führt und dies die beschriebenen Organfusionen verursacht.

Die für das Polypeptid FIDDLEHEAD kodierende Nukleinsäure ist unter der Accession Number AJ010713 bzw. 064846 (GenEMBL) zugänglich sowie in WO 98/54954 gezeigt und in der vorliegenden Anmeldung unter SEQ ID NO: 1 beschrieben.

Die Vielfalt der Elongasen, ihre Substratspezifität und ihre gewebespezifische Expression ermöglichen Pflanzen die Herstellung von unterschiedlichsten Speicher-, Kutikula- und Signallipiden. Diese Synthesevielfalt kann durch heterologe Genexpression in transgenen Pflanzen praktisch genutzt werden. Dabei kann durch Gentransfer die Syntheseleistung, die bedingt ist durch eine Elongase mit spezieller Substratspezifität, von einer Pflanzenart auf eine andere übertragen werden. Andererseits kann auch innerhalb einer Pflanzenart, z.B. durch Austausch der Promotoren, die gewebespezifische Lipidsynthese gezielt verändert werden kann. Solche Arbeiten sind in vielfältiger Weise veröffentlicht. (siehe zum Beispiel WO 96/13582, WO 98/46766, WO 98/54954, WO 95/15387, WO 00/08172).

Überraschenderweise wurde jetzt gefunden, dass mit einem herbiziden Wirkstoff aus der Oxyacetamidklasse, dem Flufenacet, der Phänotyp *fiddlehead* hervorgerufen werden kann. Der Wirkstoff phänokopiert *fiddlehead*. Darüber hinaus führt der Wirkstoff auch zum Fusionieren der Rosettenblätter.

Der Befund, dass das Oxyacetamid Flufenacet die *fiddlehead-*ähnliche Fusion von Blütenorganen hervorruft, führt zur dem Schluss, dass FIDDLEHEAD (FDH), eine Beta-Ketoacyl-CoA-Synthase, der Interaktionspartner (das Target) des herbiziden Wirkstoffes ist.

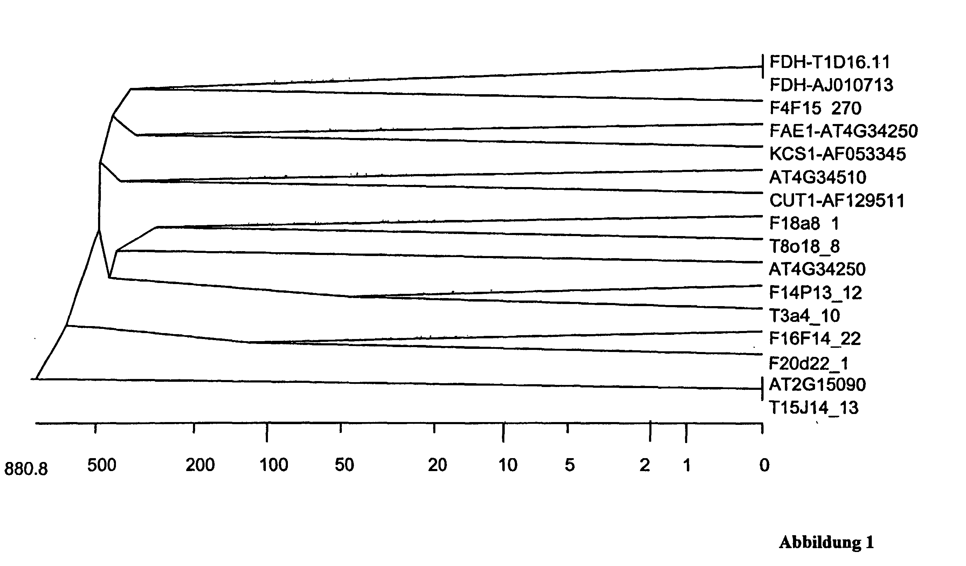

Da die Beta-Ketoacyl-CoA-Synthasen, im Besonderen auch FIDDLEHEAD aus Arabidopsis und anderen Pflanzen, starke Homologien zueinander aufweisen, können auch homologe Polypeptide, die von entsprechenden homologen Nukleinsäuren kodiert werden, sowie weitere Mitglieder der Genfamilie als molekulare Interaktionspartner (Targets) herbizider Wirkstoffe bzw. der vorstehend genannten Stoffklasse verwendet werden. Herbizide Wirkstoffe können also mit verschiedenen Beta-Ketoacyl-CoA-Synthasen interagieren, wobei die Interaktion mit den unterschiedlichen in Pflanzen vorkommenden Beta-Ketoacyl-CoA-Synthasen nicht immer gleich stark sein muss. Dies könnte unter anderem die beobachtete Selektivität der Herbizidklasse erklären. Weitere Mitglieder der Genfamilie, wie Beta-ketoacyl-CoA Synthasen aus Arabidopsis, die zu FIDDLEHEAD (FDH) aus *Arabidopsis thaliana* (Acc. Nr. AJ010713 oder 064846) homolog sind, sind beispielhaft und nicht abschließend in der nachfolgenden Tabelle sowie in Abb. 1 aufgeführt. Dort ist auch der phylogenetische Verwandtschaftsgrad der gezeigten Gene zueinander qualitativ ablesbar.

| | **Gen (*Arabidopsis thaliana*)** | **Accession Nummer** | **Beschreibung** |
|---|---|---|---|
| **1** | **T1D16.11** | tremb1\|(AC004484\|AC004484_11 | putative Beta-ketoacyl-CoA Synthase |
| **2** | **F18A8.1** | tremb1\|AC003105\|AC003105_1 | putative Beta-ketoacyl-CoA Synthase |
| **3** | **F16F14.22** | tremb1\|AC007047\|AC007047_19 | putative Beta-ketoacyl-CoA Synthase |
| **4** | **F20D22.1** | tremb1\|AC002411\|AC002411_1 | putative 3-oxoacyl-[acyl-carrier-protein] Synthase (EC 2.3.1.41) |
| **5** | **CUT1** | tremb1\|AF129511\|AF129511_1 | "very-long-chain fatty acid condensing enzyme" (CUT1) |
| **6** | **KCS1** | tremb1\|AF053345\|AF053345_1 | Fettsäureelongase 3-ketoacyl-CoA-Synthase 1-Gen ("KCS1"); KCS1-Produkt: "Fettsäure-Elongase 3-ketoacyl-CoA Synthase 1". |
| 7 | **AT4g34510** | tremblnew\|AL161585\|ATCHRIV81_35 | putative Ketoacyl-CoA Synthase |
| 8 | **T3A4.10** | tremb1\|AC005819\|AC005819_10 | putative Beta-ketoacyl-CoA Synthase |
| 9 | **AT4g34250** | tremblnew\|AL161585\|ATCHRIV81_9 | Fettsäure-Elongase-ähnliches Protein |
| 10 | **AT4g34520 (FAE 1)** | tremblnew\|AL161585\|ATCHRIV81_36 | Fettsäure-Elongase 1 (FAE 1) |
| **11** | **F14P13.12** | tremb1\|AC009400\|AC009400_12 | putative Fettsäure-Elongase 3-ketoacyl-CoA Synthase 1 |
| **12** | **F4F15.270** | tremb1\|AL049711\|ATF4F15_27 | Beta-ketoacyl-CoA Synthase-ähnliches Protein |
| 13 | **T8O18.8** | tremb1\|AC007171\|AC007171_8 | putative Fettsäure-Elongase |

In der Publikation von Böger et al. 2000 (Pest Manag. Sci. 56, 497-508) wird geschlossen, dass pflanzliche VLCFAE die primären Targets von Chloroacetamiden sind.

In der vorliegenden Anmeldung wird jedoch zum ersten Mal am Beispiel der Beta-Ketoacyl-CoA-Synthase FIDDLEHEAD gezeigt, dass Beta-Ketoacyl-CoA-Synthasen Zielproteine (Targets) für herbizide Wirkstoffe sind und zur Identifizierung neuer, verbesserter herbizider Wirkstoffe in dafür geeigneten Verfahren (Assays) eingesetzt werden können.

Dies gilt insbesondere für Polypeptide aus Pflanzen, die eine Homologie zum Polypeptid FIDDLEHEAD (FDH) aus Arabidopsis aufweisen, wobei bevorzugt Polypeptide mit einer Homologie von 80 % und ganz besonders bevorzugt von 90 % zu FDH umfasst sind.

Ganz besonders bevorzugt kann das Polypeptid FDH aus Arabidopsis zur Identifizierung von neuen herbiziden Wirkstoffen verwendet werden

Gegenstand der vorliegenden Erfindung ist deshalb die Verwendung von isolierten pflanzlichen Polypeptiden mit der biologischen Aktivität einer Beta-Ketoacyl-CoA-Synthase zum Identifizieren von Herbiziden.

Gegenstand der vorliegenden Erfindung ist ebenfalls die Verwendung von isolierten pflanzlichen Polypeptiden mit der biologischen Aktivität einer Beta-Ketoacyl-CoA-Synthase, die eine Homologie von 80 % und besonders bevorzugt von 90 % zum Polypeptid FDH aus Arabidopsis aufweisen, zum Identifizieren von Herbiziden.

Gegenstand der vorliegenden Erfindung ist ebenfalls die Verwendung von isolierten Polypeptiden aus Arabidopsis mit der biologischen Aktivität einer Beta-Ketoacyl-CoA-Synthase, die eine Homologie von 80 % und besonders bevorzugt von 90 % zum Polypeptid FDH aus Arabidopsis aufweisen, zum Identifizieren von Herbiziden.

Gegenstand der vorliegenden Erfindung ist insbesondere die Verwendung des Polypeptids FIDDLEHEAD gemäß SEQ ID NO: 2 zum Identifizieren von Herbiziden.

Gegenstand der vorliegenden Verwendung ist ebenfalls die Verwendung von für Beta-Ketoacyl-CoA-Synthasen kodierenden Nukleinsäuren zum Identifizieren von Herbiziden.

Gegenstand der vorliegenden Verwendung ist ebenfalls die Verwendung von für Beta-Ketoacyl-CoA-Synthasen kodierenden Nukleinsäuren mit einer Homologie zur Sequenz fiddlehead gemäß der kodierenden Sequenz von SEQ ID NO: 1 von 80 % und besonders bevorzugt von 90 % zum Identifizieren von Herbiziden. Besonders bevorzugt sind dabei Homologe aus Arabidopsis.

Gegenstand der vorliegenden Erfindung ist insbesondere auch die Verwendung der für das Polypeptid FIDDLEHEAD kodierenden Nukleinsäure gemäß SEQ ID NO: 1 zum Identifizieren von Herbiziden.

Bevorzugt handelt es sich bei den erfindungsgemäß zu verwendenden Polynukleotiden oder Polynukleotid-Fragmenten um solche, die Polynukleotiden aus Pflanzen entsprechen.

Besonders bevorzugt handelt es sich bei den erfindungsgemäß zu verwendenden Polynukleotiden oder Polynukleotid-Fragmenten um solche, die Polynukleotiden aus Arabidopsis entsprechen.

Ganz besonders bevorzugt umfassen die erfindungsgemäß zu verwendenden Polypeptide einer Sequenz ausgewählt aus
a) der Sequenz gemäß SEQ ID NO: 2, und
b) Sequenzen, welche eine zumindest 80 %ige, besonders bevorzugt eine 90 %ige Identität mit der unter a) definierten Sequenz aufweisen.

Eine ganz besonders bevorzugte Ausführungsform der erfindungsgemäß zu verwendenden Nukleinsäuren stellt ein cDNA-Molekül mit der für FDH kodierenden Sequenz gemäß SEQ ID NO: 1 dar, wobei die unter SEQ ID NO: 1 gezeigten Introns nicht mehr enthalten sind.

Die für FDH kodierenden Bereiche der unter SEQ ID NO: 1 gezeigten Nukleinsäure erstrecken sich von Position 176-583, 1119-1745 und 1821-2438.

Bei den erfindungsgemäß zu verwendenden Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die wie in SEQ ID NO: 1 gezeigt Introns enthalten können, und cDNAs.

Bevorzugt handelt es sich bei den erfindungsgemäß zu verwendenden Nukleinsäuren um DNA oder DNA-Fragmente, die genomischer DNA aus Pflanzen entsprechen.

Besonders bevorzugt handelt es sich bei den erfindungsgemäß zu verwendenden Nukleinsäuren um DNA oder DNA-Fragmente, die genomischer DNA von Arabidopsis entsprechen.

Ganz besonders bevorzugt umfassen die erfindungsgemäß zu verwendenden Nukleinsäuren eine Sequenz ausgewählt aus
a) der Sequenz gemäß SEQ ID NO: 1,
b) Sequenzen, die für ein Polypeptid codieren, welches die Aminosäuresequenz gemäß SEQ ID NO: 2 umfasst,
c) Sequenzen, welche eine zumindest 80 %ige, besonders bevorzugt eine 90 %ige Identität mit der unter a) definierten Sequenz aufweisen.

Eine ganz besonders bevorzugte Ausführungsform der erfindungsgemäß zu verwendenden Nukleinsäuren stellt ein cDNA-Molekül mit der für FDH kodierenden Sequenz gemäß SEQ ID NO: 1 dar.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Pflanzen als Arabidopsis isoliert werden, welche für Beta-Ketoacyl-CoA-Synthasen und insbesondere für FIDDLEHEAD kodieren, welche dieselben oder ähnliche Eigenschaften einer Beta-Ketoacyl-CoA-Synthase mit der Aminosäuresequenz gemäß SEQ ID NO: 2 aufweisen. So kann zum Beispiel die Nukleinsäuresequenz kodierend für die N-terminale, für FDH charakteristische Polypeptidsequenz verwendet werden, um dem FDH sequenziell und funktionell homologe, zum Identifizieren von neuen herbiziden Wirkstoffen geeignete Nukleinsäuresequenzen bzw. Polypeptide zu finden.

Hybridisierungsbedingungen werden nach folgender Formel näherungsweise berechnet:

Die Schmelztemperatur Tm = 81,5°C + 16,6 log[c(Na⁺)] + 0,41(% G + C)) - 500/n (Lottspeich und Zorbas, 1998).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5-15°C unterhalb Tm und einer Ionenstärke von 15 mM Na⁺ (entspricht 0.1 x SSC) gewaschen. Wird eine RNA-Probe zur Hybridisierung verwendet, so ist der Schmelzpunkt um 10-15°C höher.

### Bevorzugte Hybridisierungsbedingungen sind nachstehend angegeben:

Hybridisierungslösung: DIG Easy Hyb (Fa.: Roche) Hybridisierungstemperatur: 37°C, bevorzugt 42°C (DNA-DNA), 50°C (DNA-RNA).
1. Waschschritt: 2x SSC, 0,1 % SDS 2x5 min bei Raumtemperatur;
2. Waschschritt: 1x SSC, 0,1 % SDS 2x 15 min bei 50°C; bevorzugt 0,5x SSC, 0,1 % SDS 2x 15 min bei 65°C; besonders bevorzugt 0,2x SSC, 2x15min bei 68°C.

Der Ausdruck "aktives Zentrum" bezieht sich auf eine Peptidregion, die funktionell der Peptidregion mit einer Aminosäuresequenz von Position 191 bis Position 310 der Sequenz gemäß SEQ ID NO: 2 entspricht. Die reaktive Aminosäure Cystein befindet sich an Position 257.

Der Grad der Identität der Nukleinsäuren wird vorzugsweise bestimmt mit Hilfe des Programms NCBI BLASTN Version 2.0.4. (Altschul et al., 1997).

Der Ausdruck "regulatorische Regionen", wie er hierin verwendet wird, bezieht sich auf nicht-translatierte Regionen des betreffenden Gens, wie Promotoren, Enhancer, Repressor- oder Aktivator-Bindungsstellen oder Terminationssequenzen, die mit zellulären Proteinen interagieren, wodurch die Transkription gesteuert wird.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die T3-, T7- oder SP6-Promotoren für prokaryotische Zellen oder zellfreie Systeme, sowie gewebespezifische Promotoren aus Pflanzen, z.B. der epidermisspezifische Promotor von FIDDLEHEAD und die Promotoren anderer gewebespezifischer Elongasen.

Als Vektoren können alle in molekularbiologischen Laboratorien verwendeten Phagen, Plasmide, Phagemide, Phasmide, Cosmide, YACs, BACs, künstliche Chromosomen oder Partikel, die für einen Partikelbeschuss geeignet sind, verwendet werden.

Bevorzugte Vektoren sind pBIN (Bevan, 1984) und seine Derivate für pflanzliche Zellen, pFL61 (Minet et al., 1992) oder z.B. die p4XXprom Vektorsserie (Mumberg D., Müller R. und Funk M. 1995, Gene 156, 119-122) für Hefezellen, pBLUESCRIPT-Vektoren für bakterielle Zellen, lamdaZAP (Fa. Stratagene) für Phagen.

Der Ausdruck "Wirtszelle", wie er hierin verwendet wird, bezieht sich auf Zellen, die natürlicherweise die erfindungsgemäß zu verwendenden Nukleinsäuren nicht enthalten und zur Expression des erfindungsgemäß zu verwendenden Polypeptids geeignet sind.

Als Wirtszellen eignen sich sowohl prokaryotische Zellen, vorzugsweise *E*. *coli*, als auch eukaryotische Zellen, wie Zellen von *Saccharomyces cerevisiae*, *Pichia pastoris*, Insekten, Pflanzen, Froschoozyten und Zelllinien von Säugern.

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phosphatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen. Polypeptide, die eine gleichartige biologische Aktivität wie eine Beta-Ketoacyl-CoA-Synthase mit einer Aminosäuresequenz gemäß SEQ ID NO: 2 ausüben, werden noch als erfindungsgemäß betrachtet. Dabei müssen die erfindungsgemäß zu verwendenden Polypeptide nicht den Beta-Ketoacyl-CoA-Synthasen aus Arabidopsis entsprechen. Als erfindungsgemäß zu verwendende Polypeptide werden auch Polypeptide betrachtet, die zu Beta-Ketoacyl-CoA-Synthasen beispielsweise der folgenden Pflanzen oder zu Fragmenten davon, die noch die biologische Aktivität dieser ausüben können, homolog sind:

Dikotyle Pflanzen der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Pflanzen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Vicia.

Monokotyle Pflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Pflanzen der Gattungen: Allium, Ananas, Asparagus, Avena, Hordeum, Oryza, Panicum, Saccharum, Secale, Triticale, Triticum, Zea.

Die erfindungsgemäß zu verwendenden Polypeptide können im Vergleich zu der entsprechenden Region von natürlich vorkommenden Beta-Ketoacyl-CoA-Synthasen Deletionen oder Aminosäuresubstitutionen aufweisen, solange sie noch die biologische Aktivität der vollständigen Fettsäure-Elongasen ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg |
| Met | Leu, Tyr, Ile, Phe |
| Phe | Met, Leu, Tyr, Trp |
| Ser | Thr |
| Thr | Ser |
| Trp | Tyr, Phe |
| Tyr | Trp, Phe |
| Val | Ile, Leu |

Gegenstand der vorliegenden Erfindung ist somit auch die Verwendung von Polypeptiden, welche die biologische Aktivität einer Beta-Ketoacyl-CoA-Synthase ausüben und eine Aminosäuresequenz umfassen, die eine zumindest 80 %ige Identität, besonders bevorzugt 90 %ige Identität und ganz besonders bevorzugt 97-99 %ige Identität mit der Sequenz gemäß SEQ ID NO: 2 über deren Gesamtlänge aufweisen.

Der Grad der Identität der Aminosäuresequenzen wird vorzugsweise bestimmt mit Hilfe des Programms BLASTP + BEAUTY Version 2.0 4. (Altschul et al., 1997).

Eine bevorzugte Ausführungsform der erfindungsgemäß zu verwendenden Polypeptide ist Beta-Ketoacyl-CoA-Synthase FIDDLEHEAD (FDH) mit einer Aminosäuresequenz gemäß SEQ ID NO: 2.

Die FDH-Aminosäuresequenz gehört aufgrund ihrer Homologie (Yephremov et al. 1999) zu der Familie der "Fatty Acid Elongasen" (FAE). Das FDH-Protein besitzt im Bereich der Aminosäuren von ca. 53 bis 75 und im Bereich ca. 93 bis 115 zwei potentielle Transmembran-Domänen.

Die ersten 45-50 Aminosäuren am N-Terminus sind spezifisch für das FIDDLEHEAD Protein, dessen biologische Rolle bislang nicht völlig geklärt ist.

Der N-Terminus ist so spezifisch, dass er als Erkennungssequenz für FDH dienen kann.

Der Ausdruck "biologische Aktivität einer Beta-Ketoacyl-CoA-Synthase", wie er hierin verwendet wird, bedeutet die Fähigkeit der Biosynthese von Fettsäuren mit einer Kettenlänge von 18 und mehr C-Atomen sowie das Erkennen der Substrate Malonyl-CoA Ester und/oder Fettsäure-CoA Ester mit Kettenlängen größer/gleich C₁₈.

Die erfindungsgemäß zu verwendenden Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Die markierten Oligonukleotide können auch verwendet werden, um ausgehend von Pflanzen-mRNA hergestellte cDNA-Banken zu durchsuchen. Klone, an die die markierten Oligonukleotide hybridisieren, werden zur Isolierung der betreffenden DNA-Fragmente ausgewählt. Nach der Charakterisierung der isolierten DNA erhält man auf einfache Weise die erfindungsgemäß zu verwendenden Nukleinsäuren.

Die erfindungsgemäß zu verwendenden Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Zur Herstellung der erfindungsgemäß zu verwendenden Polypeptide, die von den erfindungsgemäßen Nukleinsäuren kodiert werden, können Wirtszellen, die erfindungsgemäß zu verwendenden Nukleinsäuren enthalten, unter geeigneten Bedingungen kultiviert werden. Die gewünschten Polypeptide können danach auf übliche Weise aus den Zellen oder dem Kulturmedium isoliert werden. Die Polypeptide können auch in *in-vitro*-Systemen hergestellt werden.

Ein schnelles Verfahren zum Isolieren der erfindungsgemäß zu verwendenden Polypeptide, die von Wirtszellen unter Verwendung einer erfindungsgemäß zu verwendenden Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, IonenaustauschChromatographie und Affinitätschromatographie.

Da Beta-Ketoacyl-CoA-Synthasen Membranproteine bzw. membranassoziierte Proteine darstellen, werden in den Reinigungsverfahren vorzugsweise Detergensextraktionen durchgeführt, beispielsweise unter Verwendung von Detergenzien, die die Sekundär- und Tertiärstrukturen der Polypeptide nicht oder nur wenig beeinflussen, wie nichtionische Detergenzien.

Die Reinigung der erfindungsgemäß zu verwendenden Polypeptide kann die Isolierung von Membranen ausgehend von Wirtszellen, die die erfindungsgemäßen Nukleinsäuren exprimieren, umfassen. Vorzugsweise exprimieren solche Zellen die erfindungsgemäß zu verwendenden Polypeptide in einer ausreichenden Kopienanzahl, so dass die Menge der Polypeptide in einer Membranfraktion mindestens 10-fach höher ist als diejenige, die in vergleichbaren Membranen von Zellen gefunden wird, die das FDH-Gen natürlicherweise exprimieren; besonders bevorzugt ist die Menge mindestens 100-fach, ganz besonders bevorzugt mindestens 1000-fach höher.

Für Testzwecke kann auch ohne eine besondere Reinigung eine Membranfraktion, insbesondere eine Mikrosomenfraktion angereichert und verwendet werden.

Die Ausdrücke "Isolierung oder Reinigung", wie sie hierin verwendet werden, bedeuten, dass die erfindungsgemäß zu verwendenden Polypeptide von anderen Proteinen oder anderen Makromolekülen der Zelle oder des Gewebes abgetrennt werden. Vorzugsweise ist eine die erfindungsgemäß zu verwendenden Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäß zu verwendenden Polypeptide können auch ohne Fusionspartner mit Hilfe von Antikörpern, die an die Polypeptide binden, affinitätsgereinigt werden.

Gegenstand der vorliegenden Erfindung sind auch Verfahren zum Auffinden von chemischen Verbindungen, die an die erfindungsgemäßen Polypeptide binden und deren Eigenschaften verändern. Aufgrund der vielfältigen Funktionen der erfindungsgemäßen Beta-Ketoacyl-CoA-Synthasen, können Modulatoren, die die Aktivität beeinflussen, neue wuchsregulierende oder herbizide Wirkstoffe darstellen. Modulatoren können Agonisten oder Antagonisten bzw. Inhibitoren oder Aktivatoren sein.

Der Ausdruck "Agonist", wie er hierin verwendet wird, bezieht sich auf ein Molekül, das die Aktivität der Beta-Ketoacyl-CoA-Synthase beschleunigt oder verstärkt.

Der Ausdruck "Antagonist", wie er hierin verwendet wird, bezieht sich auf ein Molekül, das die Aktivität der Beta-Ketoacyl-CoA-Synthasen verlangsamt oder verhindert.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können natürliche Substrate und Liganden darstellen oder strukturelle oder funktionelle Mimetika davon.

Vorzugsweise handelt es sich bei den Modulatoren um kleine organisch-chemische Verbindungen.

Die Bindung der Modulatoren an die Beta-Ketoacyl-CoA-Synthasen kann die zellulären Vorgänge auf eine Weise verändern, die zum Absterben der damit behandelten Pflanzen führt.

Weiterhin umfasst die vorliegende Erfindung Verfahren zum Auffinden von chemischen Verbindungen, welche die Expression der erfindungsgemäß zu verwendenden Polypeptide verändern. Auch solche "Expressionsmodulatoren" können neue wuchsregulierende oder herbizide Wirkstoffe darstellen. Expressionsmodulatoren können kleine organisch-chemische Moleküle, Peptide oder Antikörper sein, die an die regulatorischen Regionen der für die erfindungsgemäß zu verwendenden Polypeptide codierenden Nukleinsäuren binden. Weiterhin können Expressionsmodulatoren kleine organisch-chemische Moleküle, Peptide oder Antikörper sein, die an ein Molekül binden, welches wiederum an regulatorische Regionen der für die erfindungsgemäß zu verwendenden Polypeptide kodierenden Nukleinsäuren bindet, und dadurch deren Expression beeinflusst. Expressionsmodulatoren können auch Antisense-Moleküle sein.

Die erfindungsgemäßen Verfahren schließen Hochdurchsatz-Screening (high throughput screening; HTS) ein. Dafür können sowohl Wirtszellen als auch zellfreie Präparationen verwendet werden, die die erfindungsgemäß zu verwendenden Nukleinsäuren und/oder die erfindungsgemäß zu verwendenden Polypeptide enthalten.

Um Modulatoren aufzufinden, kann ein synthetischer Reaktionsmix (z.B. Produkte der *in vitro*-Transkription) oder ein zellulärer Bestandteil, wie eine Membran oder irgendeine andere Präparation, die die erfindungsgemäß zu verwendenden Polypeptide enthält, zusammen mit einem markierten Substrat oder Liganden der Polypeptide in Gegenwart und Abwesenheit eines Kandidatenmoleküls, das ein Agonist oder Antagonist sein kann, inkubiert werden. Die Fähigkeit des Kandidatenmoleküls die Aktivität der erfindungsgemäß zu verwendenden Polypeptide zu erhöhen oder zu hemmen, wird erkennbar an einer erhöhten oder verringerten Bindung des markierten Liganden oder an einer erhöhten oder verringerten Umsetzung des markierten Substrates. Moleküle, die gut binden und zu einer erhöhten Aktivität der erfindungsgemäßen Polypeptide führen, sind Agonisten. Moleküle, die gut binden, und die biologische Aktivität der erfindungsgemäß zu verwendenden Polypeptide hemmen, sind gute Antagonisten. Es kann sich dabei um Hemmstoffe der oben genannten herbiziden Stoffklassen handeln, ohne jedoch auf diese beschränkt zu sein.

Die Detektion der biologischen Aktivität der erfindungsgemäß zu verwendenden Polypeptide kann durch ein so genanntes Reportersystem verbessert werden. Reportersysteme in dieser Hinsicht umfassen, sind aber nicht beschränkt auf colorimetrisch markierte Substrate, die in ein Produkt umgewandelt werden oder ein Reportergen, das auf Veränderungen der Aktivität oder der Expression der erfindungsgemäß zu verwendenden Polypeptide anspricht oder andere bekannte Bindungstests.

Die Aktivität membranassoziierter Proteine kann auf eine weitere Weise vorteilhaft gemessen werden. Die funktionelle heterologe Expression solcher Proteine in *E*. *coli* ist oft schwierig oder unmöglich. In diesem Fall kann durch geeignete Klonierung (z.B. unter Nutzung geeigneter PCR Strategien) der katalytisch aktive Teil des Proteins vom membranständigen Teil des Proteins getrennt werden, sodass das Genprodukt ein lösliches Protein darstellt und leicht gereinigt werden kann. Zur Messung der Aktivität löslicher Proteine steht ein breites Repertoire von Meßmöglichkeiten zur Verfügung. Eine besonders empfindliche Messung kann z.B. unter Einsatz eines fluoreszenzmarkierten Liganden oder Substrates mittels Fluoreszenzpolarisation erfolgen.

Ein weiteres Beispiel für ein Verfahren, mit welchem Modulatoren der erfindungsgemäß zu verwendenden Polypeptide aufgefunden werden können, ist ein Verdrängungstest, bei dem man unter dafür geeigneten Bedingungen die erfindungsgemäß zu verwendenden Polypeptide und einen potenziellen Modulator mit einem Molekül, das bekanntermaßen an die erfindungsgemäß zu verwendenden Polypeptide bindet, wie einem natürlichen Substrat oder Liganden oder einem Substrat- oder Liganden-Mimetikum zusammenbringt. Das Substrat- oder Liganden-Mimetikum kann z.B. radioaktiv markiert, oder mit einem Fluoreszenz- oder Farbmarker (auch UV) versehen sein. Auch die erfindungsgemäß zu verwendenden Polypeptide selbst können in analoger Weise markiert werden, so dass man die Anzahl der Polypeptide, die an einen Liganden gebunden sind oder die eine Umsetzung mitgemacht haben, exakt bestimmen kann. Auf diese Weise lässt sich die Effektivität eines Agonisten oder Antagonisten ermessen.

Der Ausdruck "Mutagenese" wie er hierin verwendet wird, bezeichnet eine Methode zur Erhöhung der spontanen Mutationsrate und damit zur Isolierung von Mutanten. Dabei können Mutanten mit Hilfe von Mutagenen *in vivo* erzeugt werden, z. B. mit chemischen Verbindungen oder physikalischen Einflüssen, die geeignet sind, Mutationen auszulösen (z. B. Basenanaloga, interkalierende Stoffe, UV-Strahlen) Die gewünschten Mutanten können durch Selektion auf einen bestimmten Phänotyp hin erhalten werden. Die Position der Mutationen auf den Chromosomen kann relativ zu anderen, bekannten Mutationen durch Komplementations- und Rekombinationsanalysen bestimmt werden. Mutationen können auch gezielt in chromosomale oder extrachromosomale DNA eingebracht werden (*in vitro*-Mutagenese, site-directed-Mutagenese, error prone-PCR etc.).

Der Ausdruck "Mutante", wie er hierin verwendet wird, bezeichnet einen Organismus, der ein verändertes (mutiertes) Gen trägt. Eine Mutante ist definiert durch den Vergleich mit dem Wildtyp, der das unveränderte Gen trägt.

Da die erfindungsgemäß zu verwendenden VLCFAE, insbesondere das Protein FDH, eine Rolle in den Signal- und Entwicklungsvorgängen spielen, erhält man in transgenen "sense" Pflanzen oder in Pflanzen, die auf eine erhöhte Menge oder Aktivität entsprechender endogener VLCFAE oder in der Signaltransduktion damit verknüpfter Elemente gezielt selektioniert wurden, eine erhöhte Resistenz gegenüber herbizid wirkenden Verbindungen. Auch durch Mutation der Aminosäuresequenz können VLCFAE entwickelt werden, die gegenüber auf diese Proteine herbizid wirkende Verbindungen resistent sind. Überträgt und exprimiert man die Gene solcher natürlich isolierten oder synthetisch hergestellten Mutanten auf Pflanzen, insbesondere auf Kulturpflanzen wie z.B. Mais, Weizen, Gerste, Hafer, Reis, Roggen, Tomaten, Leguminosen, Kartoffelpflanzen, Lactuca sativa, Brassicaceen, Holzgewächse, Physcomitrella patens, so sind diese herbizidresistent oder durch eine erhöhte Toleranz gegenüber Herbiziden gekennzeichnet.

### Beispiele

### Beispiel 1

### Herbizidbehandlung von Arabidopsis thaliana mit Flufenacet: Erzeugung des fiddlehead Phänotyps

Arabidopsis Samen (Ecotyp Columbia Col-0), die bei 4°C gelagert wurden, wurden auf Erde ausgesät und bei 22°C ± 2°C im Langtag (16h Licht/8h Dunkel) kultiviert.

2 Wochen alte Pflanzen wurden mit 250 g/ha und 50 g/ha Flufenacet besprüht und bei 22°C ± 2°C im Langtag (16h Licht/8h Dunkel) weiterkultiviert. Zwischen Tag 7 und Tag 11 nach Flufenacet Behandlung verwachsen die Blütenorgane mit den obersten caulinen Blättern der Arabidopsispflanzen. Diese Verwachsungen ähneln sehr stark denen der "fiddlehead" Mutanten. Ab Tag 18 sehen alle Blüten wieder normal aus, da Flufenacet durch die Glutathion S Transferase Aktivität der Pflanzen abgebaut wird.

### Beispiel 2

### Isolierung der beschriebenen Nukleotidsequenz FDH

Die Isolierung der beschriebenen Nukleotidsequenz FIDDLEHEAD (FDH) kann analog Yephremov et al. 1999, The Plant Cell 11, 2187-2201, Pruitt et al. 2000, PNAS 97(3), 1311-1316 oder gemäß WO 98/54954 (Klon EL 4) erfolgen. Unter Zuhilfenahme der unter SEQ ID NO: 1 beschriebenen Sequenz kann die Sequenz auch mit dem Fachmann bekannten Methoden ohne weiteres gewonnen werden.

### Beispiel 3

### Herstellung transgener Arabidopsis-Pflanzen, die resistent sind gegenüber Wirkstoffen, die auf VLCFAE gerichtet sind

Ausgewählte, mutierte VLCFAE Genkonstrukte werden in geeignete pBIN Vektor-Konstrukte kloniert und auf geeignete Agrobakterienstämme übertragen. Die Vektorsequenzen tragen ein in Pflanzen selektionierbares Markergen, das z.B. die Resistenz gegen Kanamycin oder die Resistenz gegen das Herbizid BASTA vermittelt. Arabidopsis-Pflanzen werden angezogen und nach 4-6 Wochen, kurz vor der Blüte in eine Suspension des mit dem Vektorkonstrukt transformierten Agrobakterienstammes getaucht. Der Kontakt des pflanzlichen Gewebes mit der Bakteriensuspension kann durch Vakuuminfiltration verstärkt werden. Die so behandelten Pflanzen werden bis zu Samenreife weiterinkubiert. Die Samen werden geerntet und auf Selektionsmedium ausgelegt. Es überleben nur die Keimlinge, die das übertragene Genkonstrukt und das Markergen enthalten, die also erfolgreich transformiert wurden. Eine detaillierte Beschreibung der Methode findet sich bei Bechthold N. und Pelletier G.: *In planta Agrobacterium* mediated transformation of adult *Arabidopsis thaliana* plants by vacuum infiltration. In: Arabidopsis Protocols, edited by Martinez-Zapater J.M. und Salinas J., 1998 Humana Press ISBN 0-89603-391-0.

### Erläuterung der Abbildungen und des Sequenzprotokolls

- **Abb. 1:**: Phylogenetischer Baum von *Arabidopsis thaliana* beta-ketoacyl-CoA Synthasen mit Homologie zu FIDDLEHEAD (FDH) (DNA Star Programm MegAlign (Clustal Methode mit PAM250 "residue weight table")).
- **SEQ ID NO: 1:**: Nukleinsäuresequenz kodierend für das Polypeptid "FIDDLEHEAD" (FDH) aus *Arabidopsis thaliana*, einer Very Long Chain Fatty Acid Elongase. Es handelt sich um eine genomische Sequenz, weshalb noch Introns enthalten sind. Die kodierenden Bereiche sind angegeben.
- **SEQ ID NO:** 2:: Aminosäuresequenz des Polypeptids "FIDDLEHEAD" (FDH) aus *Arabidopsis thaliana*.

### Literatur

Abulnaja K.O. and Harwood J.L. 1991, Thiocarbamate Herbicides inhibit fatty acid elongation in a variety of monocotyledons. Phytochemistry 30(5), 1445-1447).
Altschul S.F., Madden T.L., SchafferA.A., Zhang J.Z.; Miller W. and Lipman, D.J. 1997. Gapped BLAST und PSI-BLAST generation of protein database search programs. Nucleic Acids Res. 25: 3389-3402.
Bechthold N. und Pelletier G. : *In planta Agrobacterium* mediated transformation of adult *Arabidopsis thaliana* Plants by vacuum infiltration. In: Arabidopsis Protocols, edited by Martinez-Zapater J.M. und Salinas J. , 1998 Humana Press ISBN 0-89603-391-0.
Bevan, M., Binary Agrobacterium vectors for plant transformation (1984), Nucl. Acids Res. 12 (22), 8711-8721.
Böger P., Matthes B. und Schmalfuß J. 2000, Towards the primary target of chloroacetamides - new findings pave the way. Pest Management Science 56: 497-508.
Lottspeich, F., Zorbas H. (Hrsg.). 1998. Bioanalytik. Spektrum Akademischer Verlag, Heidelberg, Berlin.
Matthes B., Schmalfuß J. and Boeger P. 1998, Chloracetamide Mode of Action II: Inhibition of Very Long Chain Fatty Acid Synthesis in Higher Plants. Z. Naturforsch. 53c, 1004-1011.
Millar A.A. and Kunst L. 1997, Very-long-chain fatty acid biosynthesis is controlled through the expression and specificity of the condensing enzyme. The Plant Journal 12(1), 121-131.
Millar A.A., Smith M.A., Kunst L. 2000, All fatty acids are not equal: discrimination in plant membrane lipids. Trends in Plant Science 5, 95-101.
Minet, M., Dufour, M.-E. and Lacroute, F. 1992. Complementation of *Saccharomyces cerevisiae* auxotrophic mutants by *Arabidopsis thaliana* cDNAs. Plant J. 2: 417-422.
Möllers C. und Albrecht S.1994, Screening herbicide effects on lipid metabolism of storage lipids by *in vitro* culture of microspore-derived embryoids of *Brassica napus*. J. Plant Physiol. 144, 376-384.
Mumberg D., Müller R. und Funk M. 1995, Yeast vectors for the controlled expression of heterologous proteins in different genetic backgrounds Gene 156, 119-122.
Pruitt R.E., Vielle-Calzada J-P., Ploense S.E., Grossniklaus U. and Lolle S.J. 2000, *FIDDLEHEAD*, a gene required to suppress epidermal cell interactions in Arabidopsis, encodes a putative lipid biosynthetic enzyme. PNAS 97(3), 1311-1316.
Puissant, C. und Houdebine, L.-M. 1990. An improvement of the single-step method of the RNA isolation by acid guanidinium thiocyanate-phenol-chloroform extraction. BioTechniques 8: 148-149.
Skoog, F., Miller, C.O. 1957. Chemical regeneration of growth and organ formation in plant tissue cultures in vitro. Symp. Soc. Exp. Biol. 11: 118-131.
WO 2000/008172
WO 95/15387
WO 96/13 582
WO 98/46766
WO 98/54954
Yephremov A., Wismann E., Huijser P., Huijser C., WellesenK, Saedler H. 1999, Characterization of the *FIDDLEHEAD* Gene of Arabidopsis reveals a link between adhesion response and cell differentiation in the epidermis. The Plant Cell 11, 2187-2201.

## Patentansprüche

1. Verfahren zum Auffinden von Herbiziden, umfassend die folgenden Schritte:
a) Inkontaktbringen eines isolierten Polypeptids mit der biologischen Aktivität einer Beta-Ketoacyl-CoA-Synthase oder einer Wirtszelle enthaltend ein heterologes Polypeptid mit der biologischen Aktivität einer Beta-Ketoacyl-CoA-Synthase mit einer chemischen Verbindung oder einem Gemisch von chemischen Verbindungen unter Bedingungen, die die Interaktion einer chemischen Verbindung mit dem Polypeptid erlauben, und
b) Bestimmen, ob die Aktivität des Polypeptids durch die chemische Verbindung verringert oder erhöht wird, und gegebenenfalls
c) Bestimmen der Verbindung, die die Aktivität des Polypeptids spezifisch verringert oder erhöht.

2. Verfahren zum Auffinden von Herbiziden, umfassend die folgenden Schritte:
a) Inkontaktbringen einer Wirtszelle enthaltend eine heterologe, für ein Polypeptid mit der biologischen Aktivität einer Beta-Ketoacyl-CoA-Synthase kodierende Nukleinsäure mit einer chemischen Verbindung oder einem Gemisch von chemischen Verbindungen,
b) Bestimmen der Polypeptidkonzentration, und
c) Bestimmen der Verbindung, welche die Expression des Polypeptids spezifisch beeinflusst.

3. Verfahren gemäß Anspruch 1, **dadurch gekennzeichnet, dass** die Polypeptide eine Sequenz umfassen, die ausgewählt ist aus
a) der Sequenz gemäß SEQ ID NO: 2,
b) Sequenzen, welche eine zumindest 80 %ige Identität mit der unter a) definierten Sequenz aufweisen.

4. Verfahren gemäß Anspruch 1 oder 3, **dadurch gekennzeichnet, dass** es sich bei der Wirtszelle um *E. coli* oder um eine Hefe-, Insekten-, Säugetier oder Pflanzenzelle handelt.

5. Verfahren gemäß Anspruch 2, **dadurch gekennzeichnet, dass** die Nukleinsäuren eine Sequenz umfassen, die ausgewählt ist aus
a) der Sequenz gemäß SEQ ID NO: 1,
b) Sequenzen, die für ein Polypeptid codieren, welches die Aminosäuresequenz gemäß SEQ ID NO: 2 umfasst,
c) Sequenzen, welche eine zumindest 80 %ige Identität mit der unter a) definierten Sequenz aufweisen.

6. Verwendung von isolierten Polypeptiden mit der biologischen Aktivität einer Beta-Ketoacyl-CoA-Synthase zum Identifizieren von herbizid wirksamen Verbindungen.

7. Verwendung von Nukleinsäuren, die für Polypeptide mit der biologischen Aktivität einer Beta-Ketoacyl-CoA-Synthase kodierer, zum Identifizieren von Herbiziden.

## Claims

1. Method for finding herbicides, comprising the following steps:
a) contacting an isolated polypetide with the biological activity of a beta-ketoacyl-CoA synthase or a host cell containing a heterologous polypeptide with the biological activity of a beta-ketoacyl-CoA synthase with a chemical compound or a mixture of chemical compounds under conditions which permit the interaction of a chemical compound with the polypeptide, and
b) determining whether the activity of the polypeptide is reduced or increased by the chemical compound, and optionally
c) determining the compound which specifically reduces or increases the activity of the polypeptide.

2. Method for finding herbicides, comprising the following steps:
a) contacting a host cell containing a heterologous nucleic acid coding for a polypeptide with the biological activity of a beta-ketoacyl-CoA synthase with a chemical compound or a mixture of chemical compounds,
b) determining the polypeptide concentration and
c) determining the compound which specifically influences the expression of the polypeptide.

3. Method according to Claim 1, **characterized in that** the polypeptides include a sequence which is selected from
a) the sequence shown in SEQ ID NO: 2,
b) sequences which have an identity of at least 80% with the sequence defined under a).

4. Method according to Claim 1 or 3, **characterized in that** the host cell is *E. coli* or a yeast, insect, mammalian or plant cell.

5. Method according to Claim 2, **characterized in that** the nucleic acids comprise a sequence which is selected from
a) the sequence shown in SEQ ID NO: 1,
b) sequences which code for a polypeptide including the amino acid sequence shown in SEQ ID NO: 2,
c) sequences which have an identity of at least 80% with the sequence defined under a).

6. Use of isolated polypeptides with the biological activity of a beta-ketoacyl-CoA synthase for identifying herbicidally active compounds

7. Use of nucleic acids which code for polypeptides with the biological activity of a beta-ketoacyl-CoA synthase for identifying herbicides.

## Revendications

1. Procédé d'identification d'herbicides, comprenant les étapes suivantes :
a) mise en contact d'un polypeptide isolé avec activité biologique d'une bêta-cétoacyl-CoA-synthétase ou d'une cellule hôte contenant un polypeptide hétérologue avec l'activité biologique d'une bêta-cétoacyl-CoA-synthétase, avec un composé chimique ou un mélange de composés chimiques dans des conditions qui permettent l'interaction d'un composé chimique avec le polypeptide, et
b) détermination de la diminution ou de l'augmentation de l'activité du polypeptide par le composé chimique, et le cas échéant
c) détermination du composé qui diminue ou augmente spécifiquement l'activité du polypeptide.

2. Procédé d'identification d'herbicides, comprenent les étapes suivantes :
a) mise en contact d'une cellule hôte contenant un acide nucléique hétérologue, codant un polypeptide avec l'activité biologique d'une bêta-cétoacyl-CoA-synthétase, avec un composé chimique ou un mélange de composés chimiques
b) détermination de la concentration du polypeptide, et
c) détermination du composé qui influence spécifiquement l'expression du polypeptide.

3. Procédé suivant la revendication 1, **caractérisé en ce que** le polypeptide comprend une séquence qui est choisie parmi :
a) la séquence selon SEQ ID N°2,
b) des séquences qui présentent une identité d'au moins 80% avec la séquence définie sous a).

4. Procédé suivant la revendication 1 ou 3, **caractérisé en ce que** la cellule hôte consiste en *E. coli* ou en une cellule de levure, d'insecte, de mammifère ou végétale.

5. Procédé suivant la revendication 2, **caractérisé en ce que** l'acide nucléique comprend une séquence, qui est choisie parmi :
a) la séquence selon SEQ ID N°1,
b) des séquences qui codent un polypeptide, qui comprend la séquence des acides aminés selon SEQ ID N°2,
c) des séquences, qui présentent une identité d'au moins 80% avec la séquence définie sous a).

6. Utilisation de polypeptides isolés avec l'activité biologique d'une bêta-cétoacyl-CoA-synthétase pour l'identification de composés actifs comme herbicides.

7. Utilisation d'acides nucléiques, qui codent des polypeptides isolés avec l'activité biologique d'une bêta-cétoacyl-CoA-synthétase pour l'identification d'herbicides.


### SEQUENZPROTOKOLL

<110> BAYER AG
<120> Verwendung von VLCFAE zum Identifizieren von herbizid wirksamen Verbindungen
<130> Le A 34 730
<140>
   <141>
<160> 2
<170> PatentIn Ver. 2.1
<210> 1
   <211> 2782
   <212> DNA
   <213> Arabidopsis thaliana
<220>
   <221> CDS
   <222> (176)..(583)
<220>
   <221> CDS
   <222> (1119)..(1745)
<220>
   <221> CDS
   <222> (1821).. (2438)
<400> 1
   acattaacta cctctcacca accaccaaac ccaatcccca caatattacc attactctca 60
   tataactaca catattcata tttacatttt ttgccaacac aactccttat aagatataca
   120
   cttcatcaac ctatagatct cactcacata atcaacctac aaaacaaaaa caaga atg
   178
                                                                Met
                                                                  1
   ggt aga tcc aac gag caa gat ctg ctc tct acc gag atc gtt aat cgt
   226
   Gly Arg Ser Asn Glu Gln Asp Leu Leu Ser Thr Glu Ile Val Asn Arg
                     5                  10                  15
   ggg atc gaa cca tcc ggt cct aac gcc ggc tca cca acg ttc tcg gtt
   274
   Gly Ile Glu Pro Ser Gly Pro Asn Ala Gly Ser Pro Thr Phe Ser Val
                20                  25                  30
   agg gtc agg aga cgt ttg cct gat ttt ctt cag tcg gtg aac ttg aag
   322
   Arg Val Arg Arg Arg Leu Pro Asp Phe Leu Gln Ser Val Asn Leu Lys
            35                  40                  45
   tac gtg aaa ctt ggt tac cac tac ctc ata aac cat gcg gtt tat ttg
   370
   Tyr Val Lys Leu Gly Tyr His Tyr Leu Ile Asn His Ala Val Tyr Leu
    50                 55                  60                  65
   gcg acc ata ccg gtt ctt gtg ctg gtt ttt agt gct gag gtt ggg agt
   418
   Ala Thr Ile Pro Val Leu Val Leu Val Phe Ser Ala Glu Val Gly Ser
                   70                  75                  80
   tta agc aga gaa gag att tgg aag aag ctt tgg gac tat gat ctt gca
   466
   Leu Ser Arg Glu Glu Ile Trp Lys Lys Leu Trp Asp Tyr Asp Leu Ala
               85                  90                  95
   act gtt atc gga ttc ttc ggt gtc ttt gtt tta acc gct tgt gtc tac
   514
   Thr Val Ile Gly Phe Phe Gly Val Phe Val Leu Thr Ala Cys Val Tyr
           100                 105                 110
   ttc atg tct cgt cct cgc tct gtt tat ctt att gat ttc gct tgt tac
   562
   Phe Met Ser Arg Pro Arg Ser Val Tyr Leu Ile Asp Phe Ala Cys Tyr
       115                 120                 125
   aag ccc tcc gat gaa cac aag gtacgtccca acttttccat agaggaaata
   613
   Lys Pro Ser Asp Glu His Lys
   130                 135
   gtctaaatta cttttaccca aaaaaaaaaa aaaaaaaaaa atctaaatta agtatactta
   673
   agaaattata attagatttg tcaaaaaata ataattataa ttagatggat tagttgttta
   733
   tagggctgcc taaataaaat aaaattttgc ctttgcatgt gttacgttag taattatttt
   793
   tcaggtatat ataaaaagta attattttgc aaaaccttta gatattggtt acgtttgatt
   853
   taaaaccgaa tggtttcgta gaaatttgag aaagtagata acctaaaaac tccgattaaa
   913
   gaaaccggtt tgacttatat aattttaact ggtttctgtt ttcattttat tttataaaaa
   973
   aaacaatcca aatttacgac ctataatcaa aggagattga taggaaccgg actgataatt
   1033
   aaatgaagct gaatcaaacc aaacaaaagt tcatttaatt ccggttctct cgggtttaat
   1093
   ctctttttgc attggattgg tttag gtg aca aaa gaa gag ttc ata gaa cta
   1145
                               Val Thr Lys Glu Glu Phe Ile Glu Leu
                                           140                 145
   gcg aga aaa tca ggg aag ttc gac gaa gag aca ctc ggt ttc aag aag
   1193
   Ala Arg Lys Ser Gly Lys Phe Asp Glu Glu Thr Leu Gly Phe Lys Lys
                   150                 155                 160
   agg atc tta caa gcc tca ggc ata ggc gac gag aca tac gtc cca aga
   1241
   Arg Ile Leu Gln Ala Ser Gly Ile Gly Asp Glu Thr Tyr Val Pro Arg
               165                 170                 175
   tcc atc tct tca tca gaa aac ata aca acg atg aaa gaa ggt cgt gaa
   1289
   Ser Ile Ser Ser Ser Glu Asn Ile Thr Thr Met Lys Glu Gly Arg Glu
           180                 185                 190
   gaa gcc tct aca gtg atc ttt gga gca cta gac gaa ctc ttc gag aag
   1337
   Glu Ala Ser Thr Val Ile Phe Gly Ala Leu Asp Glu Leu Phe Glu Lys
       195                 200                 205
   aca cgt gta aaa cct aaa gac gtt ggt gtc ctt gtg gtt aac tgt agc
   1385
   Thr Arg Val Lys Pro Lys Asp Val Gly Val Leu Val Val Asn Cys Ser
   210                 215                 220                 225
   att ttc aac ccg aca ccg tcg ttg tcc gca atg gtg ata aac cat tac
   1433
   Ile Phe Asn Pro Thr Pro Ser Leu Ser Ala Met Val Ile Asn His Tyr
                   230                 235                 240
   aag atg aga ggg aac ata ctt agt tac aac ctt gga ggg atg gga tgt
   1481
   Lys Met Arg Gly Asn Ile Leu Ser Tyr Asn Leu Gly Gly Met Gly Cys
               245                 250                 255
   tcg gct gga atc ata gct att gat ctt gct cgt gac atg ctt cag tct
   1529
   Ser Ala Gly Ile Ile Ala Ile Asp Leu Ala Arg Asp Met Leu Gln Ser
           260                 265                 270
   aac cct aat agt tat gct gtt gtt gtg agt act gag atg gtt ggg tat
   1577
   Asn Pro Asn Ser Tyr Ala Val Val Val Ser Thr Glu Met Val Gly Tyr
       275                 280                 285
   aat tgg tac gtg gga agt gac aag tca atg gtt ata cct aat tgt ttc
   1625
   Asn Trp Tyr Val Gly Ser Asp Lys Ser Met Val Ile Pro Asn Cys Phe
   290                 295                 300                 305
   ttt agg atg ggt tgt tct gcc gtt atg ctc tct aac cgt cgt cgt gac
   1673
   Phe Arg Met Gly Cys Ser Ala Val Met Leu Ser Asn Arg Arg Arg Asp
                   310                 315                 320
   ttt cgc cat gct aag tac cgt ctc gag cac att gtc cga act cat aag
   1721
   Phe Arg His Ala Lys Tyr Arg Leu Glu His Ile Val Arg Thr His Lys
               325                 330                 335
   gct gct gac gac cgt agc ttc agg tttcattcat tttggtatta attcgtttta
   1775
   Ala Ala Asp Asp Arg Ser Phe Arg
           340                 345
   caatctcttg accgacctag taactaattt tgtgtggttt ttagg agt gtg tac cag
   1832
   Ser Val Tyr Gln
   gaa gaa gat gaa caa gga ttc aag ggg ttg aag ata agt aga gac tta
   1880
   Glu Glu Asp Glu Gln Gly Phe Lys Gly Leu Lys Ile Ser Arg Asp Leu
   350 355 360 365
   atg gaa gtt gga ggt gaa gct ctc aag aca aac atc act acc tta ggt
   1928
   Met Glu Val Gly Gly Glu Ala Leu Lys Thr Asn Ile Thr Thr Leu Gly
   370 375 380
   cct ctt gtc cta cct ttc tcc gag cag ctt ctc ttc ttt gct gct ttg
   1976
   Pro Leu Val Leu Pro Phe Ser Glu Gln Leu Leu Phe Phe Ala Ala Leu
   385 390 395
   ctc cgc cga aca ttc tca cct gct gcc aaa acg tcc aca acc act tcc
   2024
   Leu Arg Arg Thr Phe Ser Pro Ala Ala Lys Thr Ser Thr Thr Thr Ser
   400 405 410
   ttc tct act tcc gcc acc gca aaa acc aat gga atc aag tct tcc tct
   2072
   Phe Ser Thr Ser Ala Thr Ala Lys Thr Asn Gly Ile Lys Ser Ser Ser
   415 420 425
   tcc gat ctg tcc aag cca tac atc ccg gac tac aag ctc gcc ttc gag
   2120
   Ser Asp Leu Ser Lys Pro Tyr Ile Pro Asp Tyr Lys Leu Ala Phe Glu
   430 435 440 445
   cat ttt tgc ttc cac gcg gca agc aaa gta gtg ctt gaa gag ctt caa
   2168
   His Phe Cys Phe His Ala Ala Ser Lys Val Val Leu Glu Glu Leu Gln
   450 455 460
   aag aat cta ggc ttg agt gaa gag aat atg gag gct tct agg atg aca
   2216
   Lys Asn Leu Gly Leu Ser Glu Glu Asn Met Glu Ala Ser Arg Met Thr
   465 470 475
   ctt cac agg ttt gga aac act tct agc agt gga atc tgg tat gag ttg
   2264
   Leu His Arg Phe Gly Asn Thr Ser Ser Ser Gly Ile Trp Tyr Glu Leu
   480 485 490
   gct tac atg gag gcc aag gaa agt gtt cgt aga ggc gat agg gtt tgg
   2312
   Ala Tyr Met Glu Ala Lys Glu Ser Val Arg Arg Gly Asp Arg Val Trp
   495 500 505
   cag atc gct ttc ggt tct ggt ttt aag tgt aac agt gtg gtg tgg aag
   2360
   Gln Ile Ala Phe Gly Ser Gly Phe Lys Cys Asn Ser Val Val Trp Lys
   510 515 520 525
   gca atg agg aag gtg aag aag cca acc agg aac aat cct tgg gtg gat
   2408
   Ala Met Arg Lys Val Lys Lys Pro Thr Arg Asn Asn Pro Trp Val Asp
   530 535 540
   tgc atc aac cgt tac cct gtg cct ctc taa attatcattc ttctaaatta
   2458
   Cys Ile Asn Arg Tyr Pro Val Pro Leu
               545                 550
   aatcaagtaa gatctctaat tactccaacc aaaagataca gtttggttgg atgataggag
   2518
   ttatttactg atcattcgta tctaagtctg ttataagaat ggatgtggct agagtcctgt
   2578
   tcagcttcaa cttgttttat tttttgtttg ttctctattg gatcttcata aactttgaga
   2638
   gattaaagaa aaaaactctt ctttagtttg atagaacaga tggtcattgt aatttcttta
   2698
   atatgtcaaa gtaaaacaat ttctttttaa ggcaatctat attcagatac ataataaatt
   2758
   tagtttacgt gtataagaag atac
   2782
<210> 2
   <211> 550
   <212> PRT
   <213> Arabidopsis thaliana
<400> 2
   Met Gly Arg Ser Asn Glu Gln Asp Leu Leu Ser Thr Glu Ile Val Asn
   1 5 10 15
   Arg Gly Ile Glu Pro Ser Gly Pro Asn Ala Gly Ser Pro Thr Phe Ser
   20 25 30
   Val Arg Val Arg Arg Arg Leu Pro Asp Phe Leu Gln Ser Val Asn Leu
   35 40 45
   Lys Tyr Val Lys Leu Gly Tyr His Tyr Leu Ile Asn His Ala Val Tyr
   50 55 60
   Leu Ala Thr Ile Pro Val Leu Val Leu Val Phe Ser Ala Glu Val Gly
   65 70 75 80
   Ser Leu Ser Arg Glu Glu Ile Trp Lys Lys Leu Trp Asp Tyr Asp Leu
   85 90 95
   Ala Thr Val Ile Gly Phe Phe Gly Val Phe Val Leu Thr Ala Cys Val
   100 105 110
   Tyr Phe Met Ser Arg Pro Arg Ser Val Tyr Leu Ile Asp Phe Ala Cys
   115 120 125
   Tyr Lys Pro Ser Asp Glu His Lys Val Thr Lys Glu Glu Phe Ile Glu
   130 135 140
   Leu Ala Arg Lys Ser Gly Lys Phe Asp Glu Glu Thr Leu Gly Phe Lys
   145 150 155 160
   Lys Arg Ile Leu Gln Ala Ser Gly Ile Gly Asp Glu Thr Tyr Val Pro
   165 170 175
   Arg Ser Ile Ser Ser Ser Glu Asn Ile Thr Thr Met Lys Glu Gly Arg
   180 185 190
   Glu Glu Ala Ser Thr Val Ile Phe Gly Ala Leu Asp Glu Leu Phe Glu
   195 200 205
   Lys Thr Arg Val Lys Pro Lys Asp Val Gly Val Leu Val Val Asn Cys
   210 215 220
   Ser Ile Phe Asn Pro Thr Pro Ser Leu Ser Ala Met Val Ile Asn His
   225 230 235 240
   Tyr Lys Met Arg Gly Asn Ile Leu Ser Tyr Asn Leu Gly Gly Met Gly
   245 250 255
   Cys Ser Ala Gly Ile Ile Ala Ile Asp Leu Ala Arg Asp Met Leu Gln
   260 265 270
   Ser Asn Pro Asn Ser Tyr Ala Val Val Val Ser Thr Glu Met Val Gly
   275 280 285
   Tyr Asn Trp Tyr Val Gly Ser Asp Lys Ser Met Val Ile Pro Asn Cys
   290 295 300
   Phe Phe Arg Met Gly Cys Ser Ala Val Met Leu Ser Asn Arg Arg Arg
   305 310 315 320
   Asp Phe Arg His Ala Lys Tyr Arg Leu Glu His Ile Val Arg Thr His
   325 330 335
   Lys Ala Ala Asp Asp Arg Ser Phe Arg Ser Val Tyr Gln Glu Glu Asp
   340 345 350
   Glu Gln Gly Phe Lys Gly Leu Lys Ile Ser Arg Asp Leu Met Glu Val
   355 360 365
   Gly Gly Glu Ala Leu Lys Thr Asn Ile Thr Thr Leu Gly Pro Leu Val
   370 375 380
   Leu Pro Phe Ser Glu Gln Leu Leu Phe Phe Ala Ala Leu Leu Arg Arg
   385 390 395 400
   Thr Phe Ser Pro Ala Ala Lys Thr Ser Thr Thr Thr Ser Phe Ser Thr
   405 410 415
   Ser Ala Thr Ala Lys Thr Asn Gly Ile Lys Ser Ser Ser Ser Asp Leu
   420 425 430
   Ser Lys Pro Tyr Ile Pro Asp Tyr Lys Leu Ala Phe Glu His Phe Cys
   435 440 445
   Phe His Ala Ala Ser Lys Val Val Leu Glu Glu Leu Gln Lys Asn Leu
   450 455 460
   Gly Leu Ser Glu Glu Asn Met Glu Ala Ser Arg Met Thr Leu His Arg
   465 470 475 480
   Phe Gly Asn Thr Ser Ser Ser Gly Ile Trp Tyr Glu Leu Ala Tyr Met
   485 490 495
   Glu Ala Lys Glu Ser Val Arg Arg Gly Asp Arg Val Trp Gln Ile Ala
   500 505 510
   Phe Gly Ser Gly Phe Lys Cys Asn Ser Val Val Trp Lys Ala Met Arg
           515                 520                 525
   Lys Val Lys Lys Pro Thr Arg Asn Asn Pro Trp Val Asp Cys Ile Asn
       530                 535                 540
   Arg Tyr Pro Val Pro Leu
   545                 550